(19) Europäisches Patentamt
European Patent Office
Office européen des brevets

(11) **EP 2 799 070 B1**

(12) **EUROPEAN PATENT SPECIFICATION**

(45) Date of publication and mention
of the grant of the patent:
**13.02.2019 Bulletin 2019/07**

(51) Int Cl.:
*A61K 31/47* [(2006.01)]    *A61K 31/337* [(2006.01)]
*A61K 31/4745* [(2006.01)]   *A61K 31/517* [(2006.01)]
*A61K 31/7068* [(2006.01)]   *A61K 45/00* [(2006.01)]
*A61P 35/00* [(2006.01)]     *A61K 31/4412* [(2006.01)]
*A61K 31/513* [(2006.01)]    *A61K 31/53* [(2006.01)]

(21) Application number: **12863476.3**

(22) Date of filing: **27.12.2012**

(86) International application number:
**PCT/JP2012/083794**

(87) International publication number:
**WO 2013/100014 (04.07.2013 Gazette 2013/27)**

(54) **EFFECT POTENTIATOR FOR ANTITUMOR AGENTS**

WIRKUNGSVERSTÄRKER FÜR ANTITUMORMITTEL

POTENTIALISATEUR D'EFFET POUR DES AGENTS ANTI-TUMORAUX

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **28.12.2011 JP 2011290125**

(43) Date of publication of application:
**05.11.2014 Bulletin 2014/45**

(73) Proprietor: **Taiho Pharmaceutical Co., Ltd.
Chiyoda-ku
Tokyo 101-8444 (JP)**

(72) Inventors:
• **FUJITA, Hidenori
Tsukuba-shi
Ibaraki 300-2611 (JP)**
• **KATO, Masanori
Tsukuba-shi
Ibaraki 300-2611 (JP)**

(74) Representative: **Blodig, Wolfgang
Wächtershäuser & Hartz
Patentanwaltspartnerschaft mbB
Weinstrasse 8
80333 München (DE)**

(56) References cited:
EP-A1- 2 287 155        WO-A1-2006/108059
WO-A1-2009/125597       WO-A1-2009/140549
WO-A2-2010/093789       US-A1- 2009 226 443

• **NISHII TAKAFUMI; YASHIRO MASAKAZU;
TANAKA HIROAKI; INOUE TORU; YAMADA
NOBUYA; YAMASHITA YOSHITO; ONODA
NAOYOSHI; MAEDA KIYOSHI; SA: "c-Met
inhibitor might be a promising
molecular-targeted molecule against for
CPT11-resistant cancer stem cells",
PROCEEDINGS OF THE ANNUAL MEETING OF
THE AMERICAN ASSOCIATION FOR CANCER
RESEARCH, vol. 50, 1 April 2009 (2009-04-01),
page 255, XP055182601, ISSN: 0197-016X**
• **CHU SHENG-HUA ET AL: "c-Met antisense
oligodeoxynucleotides increase sensitivity of
human glioma cells to paclitaxel", ONCOLOGY
REPORTS, SPANDIDOS PUBLICATIONS, GR,
vol. 24, no. 1, 1 July 2010 (2010-07-01), pages
189-194, XP009183704, ISSN: 1791-2431**
• **'c-Met Sogaizai wa Igan Kansaibo-yo SP Saibo no
Koganzai Kanjusei o Zokyo suru' JOURNAL OF
THE JAPAN SOCIETY FOR CANCER THERAPY
vol. 43, no. 2, 01 October 2008, page 390,
XP008174092**
• **'EGFR Sayoyaku irreversible EFGR-TKI ya c-Met
Sogaizai Nado tono Heiyo' MEBIO ONCOLOGY
vol. 6, no. 1, 2009, pages 23 - 31, XP008174395**
• **'ASCO Hokoku: Haigan Ryoiki' MOLECULAR
TARGETED THERAPY FOR CANCER vol. 8, no.
4, 2010, pages 267 - 270, XP008174122**
• **'HGF/c-Met o Hyoteki to shita Bunshi Chiryoyaku
Kaihatsu no Genjo' EXPERIMENTAL MEDICINE
vol. 29, no. 2, January 2011, pages 303 - 309,
XP008174130**

- 'HGF/Met Signal ni yoru EFGR-TK Sogaiyaku Taisei' RESPIRATORY MEDICINE vol. 17, no. 3, 2010, pages 283 - 288, XP008174129

- 'HGF/Met Signal ni yoru EFGR-TK Sogaiyaku Taisei' RESPIRATORY MEDICINE vol. 17, no. 3, 2010, pages 283 - 288, XP008174129

**Description**

Field of the Invention

The present invention relates to an agent for potentiating antitumor effect and an antitumor drug comprising a combination of another antitumor agent.

Background of the Invention

There are various kinds of antitumor agents, and are broadly classified into, for example, alkylating agents, platinum compounds, antimetabolite agents, topoisomerase inhibitors, microtubule inhibitors, antitumor antibiotics, molecular target drugs. Also, in recent years, antitumor agents are frequently used in combination therapy, rather than administered alone.

For example, combination therapy of 4-(3-chloro-4-(cyclopropylaminocarbonyl)aminophenoxy)-7-methoxy-6-quinolinecarboxamide, which is an angiogenesis inhibitor having a VEGF receptor kinase inhibitory activity, with Taxane has been reported (Patent Document 1).

On the other hand, c-Met is a receptor tyrosine kinase which is classified as one of proto-oncogenes. It has been reported that, when c-Met is overexpressed, mutated or translocated frequently in various kinds of cancers (for example, renal cell cancer, gastric cancer, lung cancer, colorectal cancer, pancreatic cancer, ovarian cancer, hepatic cell cancer, head and neck cancer, melanoma), activation of c-Met is enhanced, and leads to cell proliferation, infiltration/metastasis, tumor formation, neovascularization or anti-apoptosis (for example, Non-Patent Document 1). Therefore, a compound having a c-Met inhibiting effect is known as a useful antitumor agent (Patent Document 2). Combination of a quinoline derivative which is a c-Met inhibitor and is amultikinase inhibitor including c-Met andAXL, with an ErbB inhibitor, has also been reported (Patent Document 3).

Citation List

Patent Documents

Patent Document 1: WO 2009/096377
Patent Document 2: WO 2009/125597
Patent Document 3: WO 2009/137429

Non-Patent Document 1: J. Cell Biol. 111, p. 2097-2108 (1990)

Summary of the Invention

Problem to be Solved by the Invention

However, it is unknown whether, when a certain antitumor agent and another antitumor agent are used in combination, their antitumor effect is potentiated, or the effect is weakened.

An object of the present invention is to provide a combination of two kinds of antitumor agents used in combination, by which their antitumor effect is potentiated.

Means for Solving the Problem

Thus, the inventors of the present invention has selected a certain kind of c-Met inhibitor, and studied the activity in the case of using the compound and another antitumor agent in combination. The inventors have found that an acylthiourea compound having an aminocarbonyl group as a substituent at 6-position and having an alkoxy group as a substituent at 7-position of a quinoline ring, namely 4-(2-Fluoro-4-(3-(2-phenylacetyl)thioureido)phenoxy)-7-methoxy-N-methylquinoline-6-carboxamide (also referred to herein as the acylthiourea compound (I) ; or compound (1)), or a pharmaceutically acceptable salt thereof, shows strong c-Met inhibiting activity with reduced adverse side effects. And the inventors also have found that when used in combination with another antitumor agent, the acylthiourea compound or a salt thereof extends the efficacy range or the antitumor spectrum by an excellent antitumor effect potentiating activity, thus completed the present invention.

The present invention relates to the following [1] to [5].

[1] An antitumor drug comprising a combination of 4-(2-Fluoro-4-(3-(2-phenylacetyl)thioureido)phenoxy)-7-methoxy-N-methylquinoline-6-carboxamide or a pharmaceutically acceptable salt thereof, with another antitumor agent.

[2] The antitumor drug according to [1], wherein the other antitumor agent is a member selected from the group consisting of paclitaxel, gemcitabine, lapatinib, a tegafur-gimeracil-oteracil potassium combination drug, and irinotecan.

[3] 4-(2-Fluoro-4-(3-(2-phenylacetyl)thioureido)phenoxy)-7-methoxy-N-methylquinoline-6-carboxamide or a pharmaceutically acceptable salt thereof, for use in potentiating the antitumor effect of another antitumor agent.

[4] The 4-(2-fluoro-4-(3-(2-phenylacetyl)thioureido)-phenoxy)-7-methoxy-N-methylquinoline-6-carboxamide or a pharmaceutically acceptable salt thereof for the use according to [3], wherein the other antitumor agent is a member selected from the group consisting of paclitaxel, gemcitabine, lapatinib, a tegafur-gimeracil-oteracil potassium combination drug, and irinotecan.

[5] 4-(2-fluoro-4-(3-(2-phenylacetyl)thioureido)- phenoxy)-7-methoxy-N-methylquinoline-6-carboxamide or a pharmaceutically acceptable salt thereof, for use in treating a tumor by being administered together with another antitumor agent as a single dosage or as a separate dosage, wherein the other antitumor agent is a member selected from the group consisting of paclitaxel, gemcitabine, lapatinib, a tegafur-gimeracil-oteracil potassium combination drug, and irinotecan.

Effects of the Invention

The acylthiourea compound (I), namely 4-(2-fluoro-4-(3-(2-phenylacetyl)thioureido)- phenoxy)-7-methoxy-N-methylquinoline-6-carboxamide, or a pharmaceutically acceptable salt thereof potentiates the effect of various antitumor agents when used in combination with these antitumor agents. Specifically, 1) because the acylthiourea compound (I) does not potentiate most of the adverse side effects of individual agents of the antitumor agents used in combination, the compound can be used in combination at the respective maximum effect-exhibiting doses of the respective single agents, without reducing the effective doses of the antitumor agents used in combination; 2) the compound potentiates the antitumor effect of the agents used in combination, irrespective of the drug sensitivity of the antitumor agents used in combination; and 3) the antitumor effect is observed even at a low dose at which the acylthiourea compound (I) itself does not exhibit any antitumor effect. As a result, the present invention discloses a therapeutic method with high clinical usefulness, such as extension of the efficacy range for cancer treatment and enhancement of therapeutic effects.

Description of Drawings

Fig. 1 illustrates a combined effect of 200 mg/kg/day of compound (1) and paclitaxel on human lung cancer cell line NCI-H460 subcutaneously transplanted model (nude mice).

Fig. 2 illustrates the body weight change in the case of combined use of 200 mg/kg/day of compound (1) and paclitaxel on human lung cancer cell line NCI-H460 subcutaneously transplanted model (nude mice).

Fig. 3 illustrates a combined effect of 200 mg/kg/day of compound (1) and gemcitabine on human lung cancer cell line NCI-H441 subcutaneously transplantated model (nude mice).

Fig. 4 illustrates a combined effect of 50 mg/kg/day and 250 mg/kg/day of compound (1) and TS-1 on human gastric cancer cell line NUGC-4 subcutaneously transplanted model (nude rats).

Fig. 5 illustrates a combined effect of 200 mg/kg/day of compound (1) and lapatinib on human gastric cancer cell line NCI-N87 subcutaneously transplanted model (nude mice).

Fig. 6 illustrates a combined effect of 50 mg/kg/day of compound (1) and irinotecan hydrochloride on human colorectal cancer cell line HT-29 subcutaneously transplanted model (nude mice).

Fig. 7 illustrates the body weight change in the case of combined use of 50 mg/kg/day of compound (1) and irinotecan hydrochloride on human colorectal cancer cell line HT-29 subcutaneously transplanted model (nude mice).

Fig. 8 illustrates a combined effect of 12.5 mg/kg/day of compound (1) and paclitaxel on human gastric cancer cell line NUGC-4 subcutaneously transplanted model (nude mice).

Fig. 9 illustrates the body weight change in the case of combined use of 12.5 mg/kg/day of compound (1) and paclitaxel on human gastric cancer cell line NUGC-4 subcutaneously transplanted model (nude mice) .

Detailed Description of the Invention

Examples of the pharmaceutically acceptable salt of 4-(2-fluoro-4-(3-(2-phenylacetyl)thioureido)- phenoxy)-7-methoxy-N-methylquinoline-6-carboxamide, i.e. the acylthiourea compound (I), include acid addition salts with inorganic acids such as hydrochloric acid, hydrobromic acid, hydroiodic acid, sulfuric acid, nitric acid, and phosphoric acid; acid addition salts with organic acids such as formic acid, acetic acid, propionic acid, oxalic acid, malonic acid, succinic acid, fumaric acid, maleic acid, lactic acid, malic acid, citric acid, tartaric acid, carbonic acid, picric acid, methanesulfonic acid, para-toluenesulfonic acid, and glutamic acid; salts with inorganic bases such as sodium, potassium, magnesium, calcium, and aluminum; salts with organic bases such as methylamine, ethylamine, meglumine, and ethanolamine; salts with basic amino acids such as lysine, arginine, and ornithine; and ammonium salts. Furthermore, the acylthiourea compound (I) also includes enantiomers, and also includes hydrates.

The acylthiourea compound (I) that exhibits an antitumor effect potentiating activity according to the present invention can be produced by the method described in WO 2009/125597.

The acylthiourea compound (I) is an antitumor agent showing an excellent c-Met inhibitory activity and having reduced adverse side effects. However, when used in combination with one of various other antitumor agents (hereinafter, referred to as antitumor agent A), the acylthiourea compound (I) has an activity of potentiating the antitumor effect of the antitumor agent A without exhibiting significant deterioration of toxicity.

Although the antitumor agent A whose activity is potentiated by the acylthiourea compound (I) is not particularly limited, examples thereof include antitumor antibiotic substances such as doxorubicin, doxil, and epirubicin; alkylating agents such as cyclophosphamide and nimustine; platina preparations such as cisplatin, carboplatin, and oxaliplatin; pyrimidine-based antimetabolite agents such as 5-fluorouracil (5-FU), tegafur-gimeracil-oteracil potassium (TS-1, generic name: "tegafur-gimeracil-oteracil potassium combination drug" (trade name: "TS-1")), tegafur-uracil (UFT, generic name: "tegafur-uracil combination drug" (trade name: "UFT")), capecitabine, doxifluridine, 5-fluoro-2'-deoxyuridine (FdUrd), gemcitabine, and cytarabine; purine-based antimetabolite agents such as fludarabine, cladribine, and nelarabine; folic acid antimetabolite agents such as pemetrexed and methotrexate; plant alkaloid-based antitumor agents such as paclitaxel (for example, TAXOL, ABRAXANE), docetaxel, and irinotecan; low molecular weight molecular targeted drugs such as gefitinib, erlotinib, lapatinib, everolimus, and temsirolimus; and antibody molecular targeted drugs such as bevacizumab, trastuzumab, cetuximab, and rituximab. Among these, pyrimidine-based antimetabolite agents, folic acid antimetabolite agents, plant alkaloid-based antitumor agents, and low molecular weight molecular targeted drugs are preferred.

Preferred examples of the antitumor agent A whose activity is potentiated by the acylthiourea compound (I) include paclitaxel (for example, TAXOL, ABRAXANE), gemcitabine, lapatinib, a tegafur-gimeracil-oteracil potassium combination drug, andirinotecan.

Although the malignant tumor that can be treated by the acylthiourea compound (I) together with the antitumor agent A whose activity is potentiated is not particularly limited, examples include head and neck cancer, esophageal cancer, gastric cancer, colon cancer, rectal cancer, liver cancer, gall bladder/bile duct cancer, pancreatic cancer, lung cancer, breast cancer, ovarian cancer, cervical cancer, endometrial cancer, renal cancer, urinary bladder cancer, prostate cancer, testicular tumor, bone-soft tissue sarcoma, leukemia, malignant lymphoma, multiple myeloma, skin cancer, and brain tumor.

When the acylthiourea compound (I) or a pharmaceutically acceptable salt thereof is blended with the antitumor agent A, an antitumor drug having a potentiated antitumor effect is obtained. The form of such a new antitumor drug may be a single dosage form containing the acylthiourea compound (I) or a pharmaceutically acceptable salt thereof and the antitumor agent A, or may be a separate dosage form, in which a preparation containing the acylthiourea compound (I) or a pharmaceutically acceptable salt and a preparation containing the antitumor agent A are separate . Furthermore, the means of administration of a composition containing the acylthiourea compound (I) and the means of administration of a composition containing the antitumor agent A may be the same, or may be different (for example, oral administration and injection).

In the case of incorporating the acylthiourea compound (I) or a pharmaceutically acceptable salt thereof into a pharmaceutical composition, the compound is blended with a pharmacological carrier as necessary, and various forms of administration can be employed according to the purpose of prevention or treatment. Examples of the form include an oral preparation, an injectable preparation, a suppository preparation, an ointment preparation, and a patch preparation, however an oral preparation is preferred. These forms of administration can be respectively produced by formulation methods that are commonly known to those ordinarily skilled in the art.

Regarding the pharmacological carrier, various organic or inorganic carrier materials that are commonly used as materials for formulation are used, and the pharmacological carriers are incorporated as an excipient, a binder, a disintegrant, a lubricating agent and a colorant in solid preparations; and as a solvent, for example, a dissolution aid, a suspending agent, an isotonic agent, a buffering agent, a soothing agent in liquid preparations. Furthermore, if necessary, formulation additives such as an antiseptic, an antioxidant, a colorant, a sweetening agent, and a stabilizer can also be used.

When an oral solid preparation is prepared, an excipient, and if necessary, for example, a binder, a disintegrant, a lubricating agent, a colorant, a taste masking or flavoring agent may be added to the compound of the present invention, and then, for example, a tablet, a coated tablet, a granular preparation, a powder preparation, a capsule may be produced by conventional methods.

Examples of the excipient include lactose, sucrose, D-mannitol, glucose, starch, calcium carbonate, kaolin, microcrystalline cellulose, and silicic acid anhydride.

Examples of the binder include water, ethanol, 1-propanol, 2-propanol, simple syrup, a glucose solution, an $\alpha$-starch solution, a gelatin solution, D-mannitol, carboxymethyl cellulose, hydroxypropyl cellulose, hydroxypropyl starch, methyl cellulose, ethyl cellulose, shellac, calcium phosphate, and polyvinylpyrrolidone.

Examples of the disintegrant include dried starch, sodium alginate, powdered agar, sodium hydrogen carbonate, calcium carbonate, sodium lauryl sulfate, stearic acid monoglyceride, and lactose.

Examples of the lubricating agent include purified talc, sodium stearate, magnesium stearate, borax, and polyethylene glycol.

Examples of the colorant include titanium oxide and iron oxide.

Examples of the taste masking/flavoring agent include glucose, orange peel, citric acid, and tartaric acid.

If necessary, the coating or enteric coating for the purpose of sustaining the effect may be performed according to a known coating method in oral preparations. Examples of such a coating agent include hydroxypropylmethyl cellulose, ethyl cellulose, hydroxymethyl cellulose, hydroxypropyl cellulose, polyoxyethylene glycol, and Tween 80 (registered trademark).

When an oral liquid preparation is prepared, for example, a taste masking agent, a buffering agent, a stabilizer, a flavoring agent are added to the acylthiourea compound (I), and, for example, a liquid preparation for internal use, a syrup preparation, an elixir preparation can be prepared by conventional methods. In this case, examples of the taste masking/flavoring agent include those described above, examples of the buffering agent include sodium citrate, and examples of the stabilizer include tragacanth, gum arabic, and gelatin.

When an injectable preparation is prepared, for example, a pH adjusting agent, a buffering agent, a stabilizer, an isotonic agent, a topical anesthetic agent are added to the acylthiourea compound (I), and subcutaneous, intramuscular and intravenous injectable preparations can be prepared by conventional methods. Examples of the pH adjusting agent and the buffering agent in this case include sodium citrate, sodium acetate and sodium phosphate. Examples of the stabilizer include sodium pyrosulfite, EDTA, thioglycolic aid, and thiolactic acid. Examples of the topical anesthetic agent include procaine hydrochloride and lidocaine hydrochloride. Examples of the isotonic agent include sodium chloride, glucose, D-mannitol, and glycerin.

When a suppository preparation is prepared, a carrier for formulation that is known in the art, for example, polyethylene glycol, lanolin, cacao fat, or fatty acid triglyceride, is added to the compound of the present invention, a surfactant such as, for example, Tween 80 (registered trademark) are further added thereto as necessary, and then a suppository preparation can be prepared by a conventional method.

When an ointment preparation is prepared, for example, a base, a stabilizer, a moisturizing agent, a preservative that are conventionally used are blended with the compound of the present invention as necessary, and an ointment is mixed and formulated by a conventional method. Examples of the base include liquid paraffin, white petrolatum, white beeswax, octyl dodecyl alcohol, and paraffin. Examples of the preservative include methyl para-oxybenzoate, ethyl para-oxybenzoate, and propyl para-oxybenzoate.

When a patch preparation is prepared, for example, the ointment, cream, gel, paste may be applied on a conventional support by a conventional method. Regarding the support, for example, a woven fabric or nonwoven fabric made of cotton, staple fiber, or chemical fiber; or a film or a foam sheet made of soft vinyl chloride, polyethylene, polyurethane is appropriate.

The amount of the acylthiourea compound (I) that should be incorporated in the various unit dosage forms described above may be varied depending on, for example, the symptoms of the patient to which this compound shouldbe applied, or the dosage form. However, generally, the amount per unit dosage form is about 0.05 mg to 2,000 mg in an oral preparation, about 0.01 mg to 100 mg in an injectable preparation, and about 1 mg to 2,000 mg in a suppository preparation.

Furthermore, the amount of administration per day of a medicament having the above-described form of administration varies depending on, for example, the symptoms, body weight, age, gender of the patient, and thus cannot be determined in a generalized manner. However, the amount of administration per day of an adult (body weight: 50 kg) is usually about 0.05 mg to 5,000 mg, and preferably 0.1 mg to 2,000 mg, and it is preferable to administer this amount once a day, or in about two or three divided portions per day.

When a preparation containing the acylthiourea compound (I) or a salt thereof and a preparation containing the antitumor agent A are separate preparations, the respective preparations can be administered simultaneously, or one component can be administered at an arbitrary time point before or after the administration of the other component.

The proportion of administration or mixing of the acylthiourea compound (I) or a pharmaceutically acceptable salt thereof and the antitumor agent A is not particularly limited as long as the proportion is in a range capable of providing an effect of potentiating the antitumor effect, however the proportion of the acylthiourea compound (I) or a pharmaceutically acceptable salt thereof may be about 0.001 moles to 100 moles, and preferably about 0.005 moles to 50 moles, relative to 1 mole of the antitumor agent A.

For example, when the antitumor agent A is paclitaxel (for example, TAXOL, ABRAXANE), the proportion of the acylthiourea compound (I) or a pharmaceutically acceptable salt thereof is about 0.05 moles to 50 moles relative to 1 mole of paclitaxel (for example, TAXOL, ABRAXANE) . When the antitumor agent A is gemcitabine, the proportion of the acylthiourea compound (I) or a pharmaceutically acceptable salt thereof is about 0.005 moles to 5 moles relative to 1 mole of gemcitabine. When the antitumor agent A is lapatinib, the proportion of the acylthiourea compound (I) or a pharmaceutically acceptable salt thereof is about 0.01 moles to 20 moles relative to 1 mole of lapatinib. When the antitumor agent A is a tegafur-gimeracil-oteracil potassium combination drug, the proportion of the acylthiourea compound (I) or a pharmaceutically acceptable salt thereof is about 0.05 moles to 50 moles relative to 1 mole of tegafur. When the antitumor agent A is irinotecan, the proportion of the acylthiourea compound (I) or a pharmaceutically acceptable salt thereof is about 0.05 moles to 30 moles relative to 1 mole of irinotecan. When the antitumor agent A is pemetrexed, the proportion of the acylthiourea compound (I) or a pharmaceutically acceptable salt thereof is about 0.05 moles to 20 moles relative to 1 mole of pemetrexed.

Examples

Hereinafter, the present invention is described in more detail by way of Examples and Test Examples, however the present invention is not intended to be limited to these Examples . Furthermore, regarding the dose setting of various antitumor agents having their antitumor effects potentiated by the compound of the present invention as illustrated in the following Test Examples, the maximum tolerated dose (MTD) or the maximum dose that can be administered in view of properties, which have been indicated in articles and reports were used.

For antitumor agents, the dose exhibiting the maximum efficacy and the dose exhibiting toxicity are extremely close, and in order to evaluate the maximum antitumor effect of the medicament using an animal model, it is general to evaluate the effect in the vicinity of the MTD. In the following Test Examples , the MTD and the maximum effect-exhibiting dose are considered to have the same meaning.

Example 1

4-(2-Fluoro-4-(3-(2-phenylacetyl)thioureido)phenoxy)-7-metho  xy-N-methylquinoline-6-carboxamide (compound (1)) was synthesized by the method described in WO 2009/125597.

Test Example 1 Antitumor effect potentiating activity for paclitaxel

A human lung cancer cell line (NCI-H460) was transplanted into the right chest of a 5- to 6-week old male BALB/cA Jcl-nu mouse. After the tumor transplantation, the major axis (mm) and the minor axis (mm) of the tumor were measured to calculate the tumor volume (TV), and then mice were allocated to each treatment group such that the average TV's of each group would be uniform. The day on which this grouping (n = 5) was performed was designated as Day 1.

The test solution for a paclitaxel (TAXOL injection, Bristol Myers K.K.) single administration group was prepared such that the administered dose of paclitaxel would be 60 mg/kg/day. Furthermore, the test solution for a compound (1) single administration group was prepared such that the administered dose would be 200 mg/kg/day. The compound (1) was orally administered every day for 14 consecutive days from Day 1, and paclitaxel was administered on Day 1 through the mouse tail vein. In a combined administration group, 200 mg/kg/day of the compound (1) and 60 mg/kg/day of paclitaxel were administered.

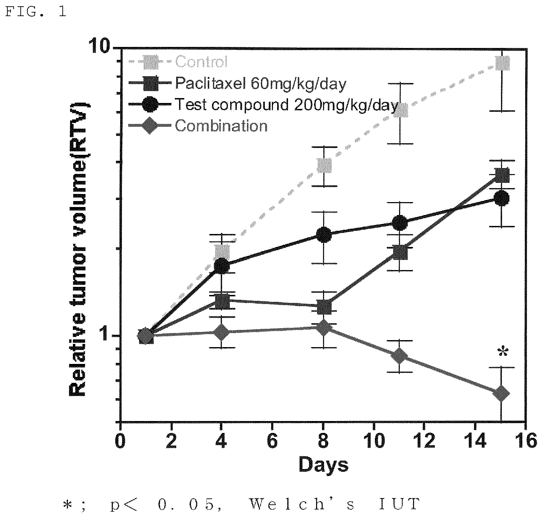

As an index of the antitumor effect, the TV on Day 15 was measured for each medicament treated group, and the relative tumor volume (RTV) for Day 1 and T/C (%) were calculated by the following formulas to evaluate the antitumor effect. For the evaluation and determination of a combined effect, when the mean RTV value of a combined administration group is statistically significantly (Welch's IUT, overall maximum $p < 0.05$) smaller than the mean RTV value of individual single administration groups, it was judged that there was a combined effect. The results are shown in Fig. 1 and Table 1. In the diagram, symbol * represents that a statistically significant difference was observed for the single administration groups.

$$TV\ (mm^3) = (Major\ axis \times minor\ axis^2)\ /\ 2$$

$$RTV = (TV\ on\ Day\ 15)\ /\ (TV\ on\ Day\ 1)$$

EP 2 799 070 B1

$$\text{T/C (\%)} = \text{(Mean RTV value of test solution administered group)} / \text{(mean RTV value of control group)} \times 100$$

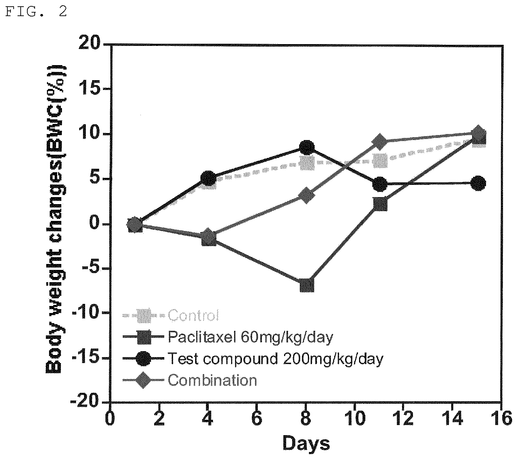

Furthermore, as an index of toxicity, the body weight [BW] was measured over time, and the mean body weight change [BWC (%)] up to Day 15 with respect to Day 1 was calculated by the following formula (n: day of body weight measurement carried out at a rate of two times/week, and the final day of measurement corresponds to Day 15, which is the final evaluation day). The results are shown in Fig. 2.

$$\text{BWC (\%)} = [\text{(BW on Day n)} - \text{(BW on Day 1)}] / \text{(BW on Day 1)} \times 100$$

[Table 1]

| Group name | Dose (mg/kg/day) | RTV (mean ± standard deviation) | T/C (%) | IUT (Welch) | | |
| --- | --- | --- | --- | --- | --- | --- |
| | | | | vs Control | vs Paclitaxel | vs Test compound |
| Control | - | 8.99 ± 2.86 | 100 | - | - | - |
| Paclitaxel | 60 | 3.67 ± 0.42 | 41 | 0.0011 | - | - |
| Test compound | 200 | 3.02 ± 0.63 | 34 | 0.00055 | - | - |
| Combination | 200 + 60 | 0.63 ± 0.15 | 7 | 0.000031 | 0.0000021 | 0.00020 |

Test Example 2 Antitumor effect potentiating activity for gemcitabine hydrochloride

A human lung cancer cell line (NCI-H441) was transplanted into the right chest of a 5- to 6-week old male BALB/cA Jcl-nu mouse, and the mouse was used in the same manner as described in Test Example 1.

The test solution for a gemcitabine hydrochloride (GEMZAR, manufactured by Eli Lilly and Co.) single administration group was prepared such that the administered dose would be 240 mg/kg/day. Furthermore, the test solution of the compound (1) was prepared such that the administered dose would be 200 mg/kg/day.

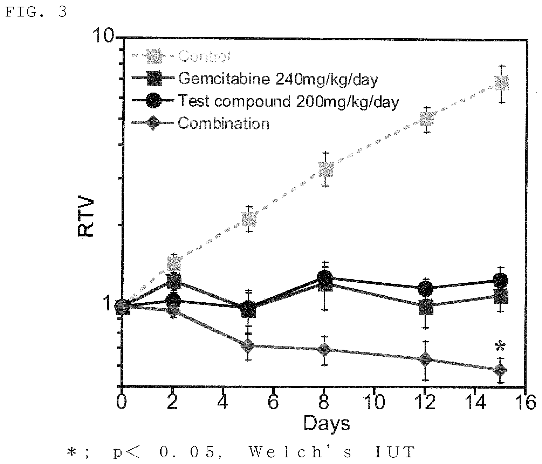

The compound (1) was orally administered every day for 14 consecutive days from Day 1, and gemcitabine hydrochloride was administered on Day 1 and Day 8 through the mouse tail vein. In a combined administration group, 200 mg/kg/day of the compound (1) and 240 mg/kg/day of gemcitabine hydrochloride were administered, and the effects were evaluated in the same manner as described in Test Example 1. The results are shown in Fig. 3 and Table 2. In the diagram, symbol * represents that a statistically significant difference was observed for the single administration groups.

[Table 2]

| Group name | Dose (mg/kg/day) | RTV (mean ± standard deviation) | T/C (%) | IUT (Welch) | | |
| --- | --- | --- | --- | --- | --- | --- |
| | | | | vs Control | vs Gemcitabine | vs Test compound |
| Control | - | 6.92 ± 1.05 | 100 | - | - | - |
| Gemcitabine hydrochloride | 240 | 1.10 ± 0.13 | 16 | 0.00020 | - | - |
| Test compound | 200 | 1.26 ± 0.15 | 18 | 0.00020 | - | - |
| Combination | 200 + 240 | 0.58 ± 0.06 | 8 | 0.00020 | 0.000200 | 0.00020 |

Test Example 3 Antitumor effect potentiating activity for TS-1

A human gastric cancer cell line (NUGC-4) was transplanted into the right chest of a 5- to 6-week old male nude rat, and the rat was used in the same manner as described in Test Example 1. The test solution for a TS-1 (TS-1, manufactured by Taiho Pharmaceutical Co., Ltd.) single administration group was prepared such that the amount of administration of tegafur would be 18 mg/kg/day. Furthermore, the test solution of the compound (1) was prepared such that the amount of administration would be 250 mg/kg/day and 50 mg/kg/day.

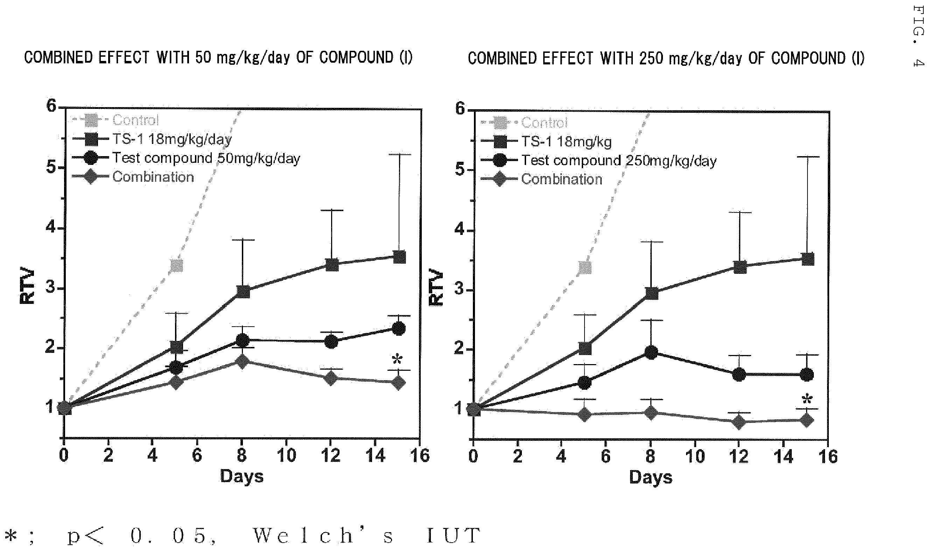

The compound (1) and TS-1 were orally administered every day for 14 consecutive days from Day 1. In a combined administration group, 250 mg/kg/day and 50 mg/kg/day of the compound (1) and 18 mg/kg/day of TS-1 were administered, and the effects were evaluated in the same manner as described in Test Example 1. The results are shown in Fig. 4 and Table 3.

[Table 3]

| Group name | Dose (mg/kg/day) | RTV (mean ± standard deviation) | | | T/C (%) | IUT (Welch) | | | |
|---|---|---|---|---|---|---|---|---|---|
| | | | | | | vs Control | vs TS-1 | vs Test Compound 1 (50 mg/kg/day) | vs Test compound 1 (250 mg/kg/day) |
| Control | - | 16.77 | ± | 15.01 | 100 | - | - | - | - |
| TS-1 | 18 | 3.55 | ± | 1.69 | 21 | 0.0028006 | - | - | - |
| Test compound 1 | 50 | 2.36 | ± | 0.44 | 14 | 0.001204 | - | - | - |
| Combination | 50 + 18 | 1.45 | ± | 0.56 | 9 | 0.000630 | 0.0475 | 0.0221 | - |
| Test compound 1 | 250 | 1.60 | ± | 0.33 | 10 | 0.000704 | - | - | - |
| Combination | 250 + 18 | 0.83 | + | 0.20 | 5 | 0.000406 | 0.0224 | - | 0.0034 |

Test Example 4 Antitumor effect potentiating activity for lapatinib

A human gastric cancer cell line (NCI-N87) was transplanted into the right chest of a 5- to 6-week old male BALB/cA Jcl-nu mouse, and the mouse was used in the same manner as described in Test Example 1. The test solution for a lapatinib (manufactured by LC Laboratories, Inc.) single administration group was prepared such that the amount of administration would be 100mg/kg/day. Furthermore, the test solution of the compound (1) was prepared such that the amount of administration would be 200 mg/kg/day of the compound (1).

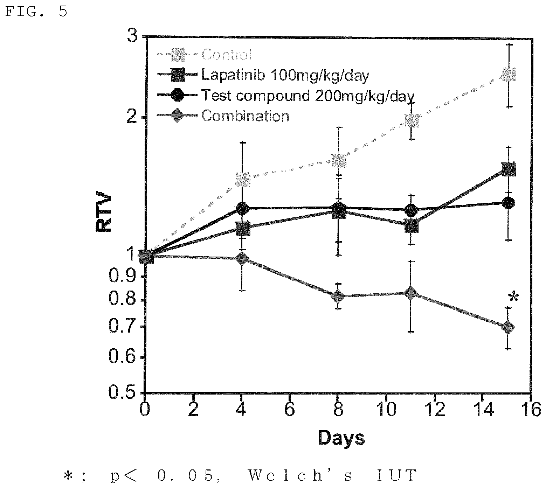

The compound (1) and lapatinib were orally administered every day for 14 consecutive days from Day 1. In a combined administration group, 200 mg/kg/day of the compound (1) and 100 mg/kg/day of lapatinib were administered, and the effects were evaluated in the same manner as described in Test Example 1. The results are shown in Fig. 5 and Table 4. In the diagram, symbol * represents that a statistically significant difference was observed for each single administration group.

[Table 4]

| Group name | Dose (mg/kg/day) | RTV (mean $\pm$ standard deviation) | T/C (%) | IUT (Welch) | | |
|---|---|---|---|---|---|---|
| | | | | vs Control | vs Lapatinib | vs Test compound |
| Control | - | 2.52 $\pm$ 0.39 | 100 | - | - | - |
| Lapatinib | 100 | 1.56 $\pm$ 0.18 | 62 | 0.0010 | - | - |
| Test compound | 200 | 1.31 $\pm$ 0.23 | 52 | 0.00020 | - | - |
| Combination | 200 + 100 | 0.70 $\pm$ 0.07 | 28 | 0.00010 | 0.000022 | 0.00080 |

Test Example 5 Antitumor effect potentiating activity for irinotecan hydrochloride

A human colorectal cancer cell line (HT-29) was transplanted into the right chest of a 5- to 6-week old male BALB/cA Jcl-nu mouse, and the mouse was used in the same manner as described in Test Example 1.

The test solution for an irinotecan hydrochloride (CAMPTO injection, manufactured by Yakult Honsha Co., Ltd.) single administration group was prepared such that the amount of administration would be 50 mg/kg/day.

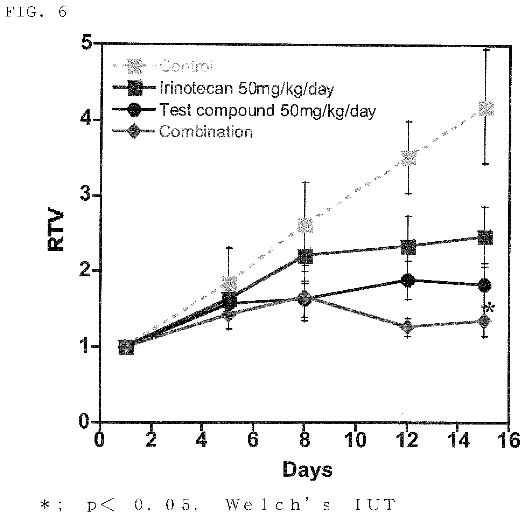

The test solution of the compound (1) was prepared such that the amount of administration would be 50 mg/kg/day of the compound (1). In a combined administration group, 50 mg/kg/day of the compound (1) and 50 mg/kg/day of irinotecan hydrochloride were administered. The compound (1) was orally administered every day for 14 consecutive days from Day 1, and irinotecan hydrochloride was administered on Day 1, Day 5, and Day 9 through the mouse tail vein, and the effects were evaluated in the same manner as described in Test Example 1. The results are shown in Fig. 6 and Table 5. In the diagram, symbol * represents that a statistically significant difference was observed for each single administration group.

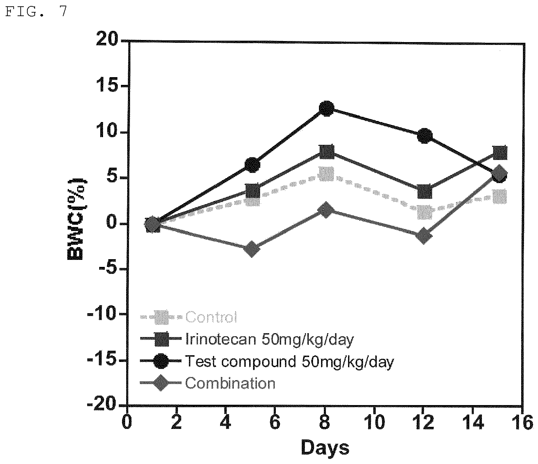

Furthermore, the body weight changes over time, which is an indicator of toxicity, were also evaluated in the same manner as described in Test Example 1. The results are shown in Fig. 7.

[Table 5]

| Group name | Dose (mg/kg/day) | RTV (mean $\pm$ standard deviation) | T/C (%) | IUT (Welch) | | |
|---|---|---|---|---|---|---|
| | | | | vs Control | vs Irinotecan hydrochloride | vs Test compound |
| Control | - | 4.19 $\pm$ 0.76 | 100 | - | - | - |
| Irinotecan hydrochloride | 50 | 2.47 $\pm$ 0.40 | 59 | 0.0079 | - | - |
| Test compound | 50 | 1.83 $\pm$ 0.29 | 44 | 0.0079 | - | - |
| Combination | 50 + 50 | 1.35 $\pm$ 0.20 | 32 | 0.0079 | 0.0079 | 0.032 |

Test Example 6 Antitumor effect potentiating activity for paclitaxel

A human gastric cancer cell line (NUGC-4) was transplanted into the right chest of a 5- to 6-week old male

EP 2 799 070 B1

BALB/cA Jcl-nu mouse, and the mouse was used in the same manner as described in Test Example 1.

The test solution for a paclitaxel (TAXOL injection, manufactured by Bristol Myers K.K.) single administration group was prepared such that the amount of administration of paclitaxel would be 60 mg/kg/day.

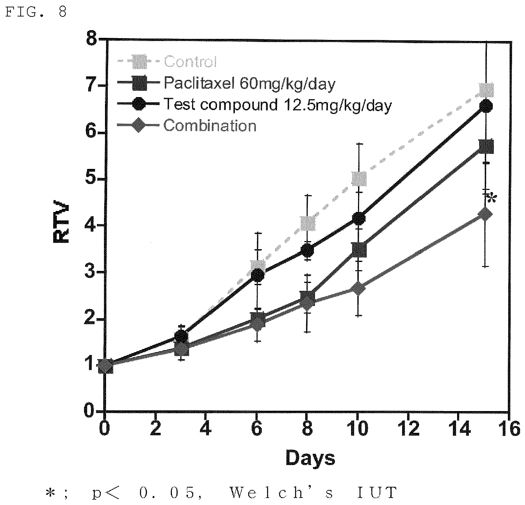

The test solution of the compound (1) was prepared such that the amount of administration would be 12.5 mg/kg/day of the compound (1). In a combined administration group, 12.5 mg/kg/day of the compound (1) and 60 mg/kg/day of paclitaxel were administered. The compound (1) was orally administered every day for 14 consecutive days from Day 1, paclitaxel was administered on Day 1 through the mouse tail vein, and evaluation was made in the same manner as described in Test Example 1. The results are shown in Fig. 8 and Table 6. In the diagram, symbol * represents that a statistically significant difference was observed for each single administration group.

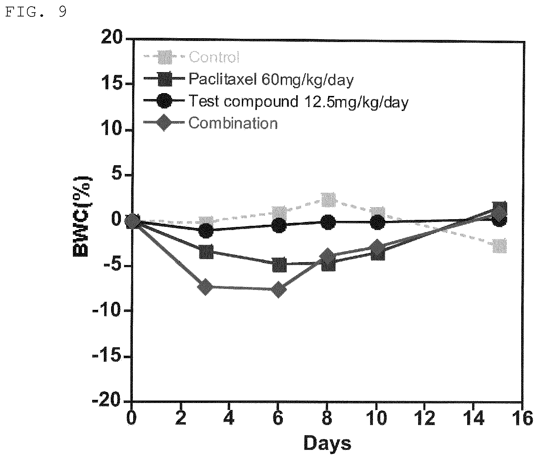

Furthermore, the body weight changes over time, which is an indicator of toxicity, were also evaluated in the same manner as described in Test Example 1. The results are shown in Fig. 9.

[Table 6]

| Group name | Dose (mg/kg/day) | RTV (mean ± standard deviation) | T/C (%) | IUT (Welch) | | |
| --- | --- | --- | --- | --- | --- | --- |
| | | | | vs Control | vs Paclitaxel | vs Test compound |
| Control | - | 6.99 ± 1.60 | 100 | - | - | - |
| Paclitaxel | 60 | 5.77 ± 0.96 | 83 | 0.1463 | - | - |
| Test compound | 12.5 | 6.63 ± 1.91 | 95 | 0.7281 | - | - |
| Combination | 12.5 + 60 | 4.27 ± 1.12 | 61 | 0.0078 | 0.032 | 0.031 |

As is obvious from Figs. 1, 3, 4, 5, 6 and 8, the acylthiourea compound (I) or a pharmaceutically acceptable salt thereof markedly potentiated the antitumor effect of various antitumor agents. The effect was observed from 12.5 mg/kg/day of the acylthiourea compound (I), which is a low dose of the compound (a dose that does not exhibit an antitumor effect, Fig. 8, Table 6), and at 200 mg/kg/day, which is a high dose (maximum effect exhibiting dose) in nude mice, and at 250 mg/kg/day, which is a high dose (maximum effect exhibiting dose) in a nude rat, remarkable shrinkage of tumors was induced by using the acylthiourea compound (I) in combination (Tables 1, 2, 3 and 4). Furthermore, aggravation of body weight reduction was not observed in combined administration (Figs. 2, 7, and 9). From these, it can be seen that an increase in the efficacy range to at least 16 times or more is secured by combined use of the compound of the present invention and various antitumor agents.

Furthermore, for example, according to comparison of Fig. 1 and Fig. 8, in the case of paclitaxel, even if the same amount of administration is used, differences in the antitumor effect (drug sensitivity) are observed depending on tumors; when paclitaxel was used in combination with the acylthiourea compound (I), potentiation of the effect is observed in both cases. That is, even for a tumor which is less susceptible to paclitaxel which is a drug for combination, when paclitaxel is used in combination with the acylthiourea compound (I), it is expected to potentiate a tumor proliferation suppressing effect of paclitaxel. This implies that combined use with the acylthiourea compound (I) expands the antitumor spectrum of other antitumor agents.

Furthermore, as shown in Fig. 2, in regard to the combined use of the acylthiourea compound (I) and various antitumor agents, since there was no significant difference in body weight reduction as compared with single administration of antitumor agents even in the case of using a high dose of 200 mg/kg/day, it was suggested that toxicity was not potentiated.

That is, it was recognized that the combined administration of the acylthiourea compound (I) or a pharmaceutically acceptable salt thereof and various antitumor agents exhibits a combination effect without exhibiting significant aggravation of toxicity, and expands the therapeutic effect range or the antitumor spectrum. Furthermore, the amount of administration of the acylthiourea compound (I) or a pharmaceutically acceptable salt is desirably set to about 0.005 moles to 50 moles relative to 1 mole of the other antitumor agent.

**Claims**

1. An antitumor drug comprising a combination of 4-(2-fluoro-4-(3-(2-phenylacetyl)thioureido)phenoxy)-7-methoxy-N-methylquinoline-6-carboxamide or a pharmaceutically acceptable salt thereof and another antitumor agent.

2. The antitumor drug according to claim 1, wherein the other antitumor agent is a member selected from the group consisting of paclitaxel, gemcitabine, lapatinib, a tegafur-gimeracil-oteracil potassium combination drug, and irinotecan.

3. 4-(2-fluoro-4-(3-(2-phenylacetyl)thioureido)phenoxy)-7-methoxy-N-methylquinoline-6-carboxamide or a pharmaceutically acceptable salt thereof for use in potentiating the antitumor effect of another antitumor agent.

4. The 4-(2-fluoro-4-(3-(2-phenylacetyl)thioureido)-phenoxy)-7-methoxy-N-methylquinoline-6-carboxamide or a pharmaceutically acceptable salt thereof for the use according to claim 3, wherein the other antitumor agent is a member selected from the group consisting of paclitaxel, gemcitabine, lapatinib, a tegafur-gimeracil-oteracil potassium combination drug, and irinotecan.

5. 4-(2-fluoro-4-(3-(2-phenylacetyl)thioureido)phenoxy)-7-methoxy-N-methylquinoline-6-carboxamide or a pharmaceutically acceptable salt thereof for use in treating a tumor by being administered together with another antitumor agent as a single dosage form or as separate dosage forms, wherein the other antitumor agent is a member selected from the group consisting of paclitaxel, gemcitabine, lapatinib, a tegafur-gimeracil-oteracil potassium combination drug, and irinotecan.

## Patentansprüche

1. Ein Antitumorarzneimittel umfassend eine Kombination von 4-(2-Fluoro-4-(3-(2-phenylacetyl)thioureido)phenoxy)-7-methoxy-N-methylquinolin-6-carboxamid oder eines pharmazeutisch akzeptablen Salzes davon und eines anderen Antitumormittels.

2. Das Antitumorarzneimittel gemäß Anspruch 1, wobei das andere Antitumormittel ein Mitglied ist, ausgewählt aus der Gruppe bestehend aus Paclitaxel, Gemcitabin, Lapatinib, einem Tegafur-Gimeracil-Oteracil-Kalium-Kombinationswirkstoff und Irinotecan.

3. 4-(2-Fluoro-4-(3-(2-phenylacetyl)thioureido)phenoxy)-7-methoxy-N-methylquinolin-6-carboxamid oder ein pharmazeutisch akzeptables Salz davon zur Verwendung zur Verstärkung des Antitumoreffekts eines anderen Antitumormittels.

4. Das 4-(2-fluoro-4-(3-(2-phenylacetyl)thioureido)-phenoxy)-7-methoxy-N-methylquinolin-6-carboxamid oder ein pharmazeutisch akzeptables Salz davon zur Verwendung gemäß Anspruch 3, wobei das andere Antitumormittel ein Mitglied ist ausgewählt aus der Gruppe bestehend aus Paclitaxel, Gemcitabin, Lapatinib, einem Tegafur-Gimeracil-Oteracil-Kalium-Kombinationswirkstoff und Irinotecan.

5. 4-(2-fluoro-4-(3-(2-phenylacetyl)thioureido)phenoxy)-7-methoxy-N-methylquinolin-6-carboxamid oder ein pharmazeutisch akzeptables Salz davon zur Verwendung bei der Behandlung eines Tumors durch Verabreichung zusammen mit einem anderen Antitumormittel als Einzeldosisform oder als separate Dosierungsformen, wobei das andere Antitumormittel ein Mitglied ist, ausgewählt aus der Gruppe bestehend aus Paclitaxel, Gemcitabin, Lapatinib, einem Tegafur-Gimeracil-Oteracil-Kalium Kombinationswirkstoff und Irinotecan.

## Revendications

1. Médicament antitumoral comprenant une combinaison de 4-(2-fluoro-4-(3-(2-phénylacétyl)-thiouréido)phénoxy)-7-méthoxy-N-méthylquinoline-6-carboxamide ou d'un sel pharmaceutiquement acceptable de celui-ci et d'un autre agent antitumoral.

2. Médicament antitumoral selon la revendication 1, dans lequel l'autre agent antitumoral est un membre choisi dans l'ensemble constitué par le paclitaxel, la gemcitabine, le lapatinib, un médicament combiné de tégafur-giméracil-otéracil potassique, et l'irinotécan.

3. 4-(2-fluoro-4-(3-(2-phénylacétyl)thiouréido)-phénoxy)-7-méthoxy-N-méthylquinoline-6-carboxamide ou un sel pharmaceutiquement acceptable de celui-ci, pour une utilisation dans la potentialisation de l'effet antitumoral d'un autre agent antitumoral.

**4.** 4-(2-fluoro-4-(3-(2-phénylacétyl)thiouréido)-phénoxy)-7-méthoxy-N-méthylquinoline-6-carboxamide ou un sel pharmaceutiquement acceptable de celui-ci, pour une utilisation selon la revendication 3, dans lequel l'autre agent antitumoral est un membre choisi dans l'ensemble constitué par le paclitaxel, la gemcitabine, le lapatinib, un médicament combiné de tégafur-giméracil-otéracil potassique, et l'irinotécan.

**5.** 4-(2-fluoro-4-(3-(2-phénylacétyl)thiouréido)-phénoxy)-7-méthoxy-N-méthylquinoline-6-carboxamide ou un sel pharmaceutiquement acceptable de celui-ci, pour une utilisation dans le traitement d'une tumeur par administration conjointement avec un autre agent antitumoral en une seule forme posologique ou en des formes posologiques séparées, dans lequel l'autre agent antitumoral est un membre choisi dans l'ensemble constitué par le paclitaxel, la gemcitabine, le lapatinib, un médicament combiné de tégafur-giméracil-otéracil potassique, et l'irinotécan.

FIG. 1

*; p< 0.05, Welch's IUT

FIG. 2

FIG. 3

*; p< 0.05, Welch's IUT

COMBINED EFFECT WITH 50 mg/kg/day OF COMPOUND (I)

COMBINED EFFECT WITH 250 mg/kg/day OF COMPOUND (I)

*; p< 0.05, Welch's IUT

FIG. 4

EP 2 799 070 B1

18

FIG. 5

*; p< 0.05, Welch's IUT

FIG. 6

*; p< 0.05, Welch's IUT

FIG. 7

FIG. 8

*; p< 0. 05, Welch's IUT

FIG. 9

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009096377 A
- WO 2009125597 A

- WO 2009137429 A

**Non-patent literature cited in the description**

- *J. Cell Biol.,* 1990, vol. 111, 2097-2108